# EUROPEAN PATENT SPECIFICATION

(11) **EP 4 353 158 B1**
(45) Date of publication and mention of the grant of the patent: **26.02.2025**
(21) Application number: 23197676.2
(22) Date of filing: 15.09.2023
(51) Int. Cl.: A61B 17/122, A61B 17/00

(54) **LOCKING FEATURE FOR EPISTAXIS CLIP**
VERRIEGELUNGSVORRICHTUNG FÜR EPISTAXIS-CLIP
ÉLÉMENT DE VERROUILLAGE POUR PINCE D'ÉPISTAXIS

(30) Priority: 14.10.2022 US 202263416186 P; 06.09.2023 US 202318462007
(43) Date of publication of application: 17.04.2024
(73) Proprietor: Freudenberg Medical, LLC, Carpinteria, CA 93013 (US)
(72) Inventor: Starkweather, Jennifer, Fort Myers, 33919 (US); Subramanya, Tejasvi, San Diego, 92122 (US); Vaughn, Talia, Santa Barbara, 93101 (US); Rogers, Theresa, Santa Barbara, 93110 (US); Nazaroff, Evan, Santa Barbara, 93101 (US)
(74) Representative: Schaumburg und Partner Patentanwälte mbB

(56) References cited:
- CN-Y- 201 160 872
- US-A- 4 457 756
- US-A- 5 464 413
- US-A1- 2003 093 113
- US-A1- 2004 010 283
- US-A1- 2005 240 219
- US-A1- 2008 015 484
- US-B2- 10 687 822

## Description

### FIELD

The present disclosure relates to a locking feature for an epistaxis clip.

### BACKGROUND

Documents US 5,464,413 A; US 2004/010283 A1; US 4,457,756 A; US 2003/093113 A1; US 2005/240219 A1 and US 2008/015484 A1 each disclose a clip comprising a pair of arms connected to one other by a hinge, each pair of arms includes a ring at a first end and a pinch tab at a second end. A pair of absorbent pads is supported by the ring of each of the pair of arms.

Document US 10 687 822 B2 discloses a hemostatic clip which includes opposing first and second clip arms connected at a shared elastic normally-opened joint portion. The clip arms include outer and inner members having tissue contacting surfaces and gaps for elastically deforming and/or shifting to apply compression and clamping forces to bodily organ or tissue. Document CN 201 160 872 Y discloses a hemostatic forceps comprising a forceps body, forceps handles and a hemostatic end, wherein the forceps body is crosswise and movably connected with the forceps handles.

Anterior epistaxis originates at the Kiesselbach's Plexus and accounts for between 90 and 95 percent of all nosebleeds. Anterior epistaxis is treated with compression and absorption. Many medical providers incorrectly treat anterior epistaxis, resulting in unnecessary and prolonged treatment and increased healthcare utilization. In particular, current treatment options and devices result in increased pain upon removal and re-aggravation of the wound and decreased cosmetic appeal. Additionally, some of the current treatment options also require pre-treatment or soaking, and completely occlude the nasal passages resulting in decreased or elimination of nasal breathing. Accordingly, it is desirable to provide an improved device for treating anterior epistaxis.

### SUMMARY

The object is solved in each case by means of an epistaxis clip having the features of claim

Advantageous embodiments are indicated in the dependent claims.

An epistaxis clip is a molded plastic clip consisting of two separate paddle-shaped wings connected through a hinge mechanism. Each wing is inserted into a nostril. The posterior displacement is limited by the anterior bridge which is formed when the two wings are connected to each other, and remains exterior to the nose at the columella. The hinge mechanism makes it easy for each wing to be inserted into the nostrils, since wings can move freely relative to each other. The hinge mechanism can be achieved through a dowel pin, a slip fit design, or through a heat staking process. The clip contains an anterior locking mechanism to favor medial compression at the septum, specifically Kiesselbach's Plexus of at least 20 mmHg. The locking feature can also be modified to include multiple pressure modes; i.e., when the locking mechanism is pressed further, it engages with another capturing feature to apply more pressure on the bleeding site (similar to a hemostat). Each wing is lined with either a single layer or a combination of polyurethane foam, PVA and/or non-woven foam for blood absorption. The chemistry of these materials can be modified to increase the absorption capacity by impregnating non-woven foams with sodium polyacrylate, can be made non-stick by coating polyurethane foam with silicone, and can be made to reduce infection risk by adding anti-microbial agents including, but not limited to, silver oxide. As the absorption material expands with absorption of blood, additional pressure is provided to the specific site of the bleed, providing absorption and additional direct pressure. When bleeding has ceased, the epistaxis clip can be unlocked and removed from the nasal passages without fear of adhering to wound or mucosa and discarded.

According to research, most medical providers incorrectly treat anterior epistaxis, resulting in unnecessary and prolonged treatment and increased healthcare utilization. Current treatment options (devices) result in increased pain upon removal and re-aggravation of the wound and decreased cosmetic appeal. They also require pre-treatment or soaking, and completely occlude the nasal passages resulting in decreased or elimination of nasal breathing. Also, current treatment/device options are more expensive relative to the proposed cost/price of the epistaxis clip. The epistaxis clip provides appropriate pressure at the physiologically appropriate location while being cost effective, cosmetically attractive, and preserving nasal function.

The epistaxis clip includes a pair of wings connected to one another by a hinged bridge portion. The pair of wings define a pair of opposing recesses and a pair of absorbent pads are disposed in the respective opposing recesses of the pair of wings. The epistaxis clip is designed to apply predetermined pressure on the Kiesselbach's plexus. The pair of absorbent pads can include an absorbent foam that resists sticking to the wound.

According to an aspect of the present disclosure, an epistaxis clip includes a pair of arms connected to one another by a hinge, each pair of arms include a wing at a first end and a pinch tab at a second end and a pair of absorbent pads are supported by the wing of each of the pair of arms.

According to a further aspect, a locking mechanism for securing the wings in a clamped position.

According to a further aspect, the locking mechanism includes a latching arm extending from a first of the pinch tabs.

According to a further aspect, the latching arm has a latch that engages a ledge of a second of the pinch tabs.

According to a further aspect, the latching arm includes a beveled surface on a distal end thereof.

According to a further aspect, the ledge is adjacent to an opening in the second one of the pinch tabs.

According to a further aspect, the ledge is on an edge of the second one of the pinch tabs.

According to a further aspect, each of the arms include a latching arm that includes a plurality of teeth, wherein the plurality of teeth of each arm engage the plurality of teeth of the other latching arm.

According to a further aspect, the arms include a curved body connecting the wing to the pinch tab.

According to a further aspect, the hinge includes a hinge pin extending through the pair of arms.

According to a further aspect, a first one of the pinch tabs include a pin thereon and a second one of the pinch tabs include a flap with an aperture, the flap being pivotal to receive the pin in the aperture.

According to a further aspect, the flap is connected to the second one of the pinch tabs by a living hinge.

According to a further aspect, the pair of arms each include a protruding hinge portion between the wing and the pinch tab, the protruding hinge portions each receiving a hinge pin extending therethrough.

According to a further aspect, a resilient member engaged with the pair of arms for biasing the wings to a clamped position.

According to a further aspect, the resilient member is disposed between the pinch tabs.

According to a further aspect, the resilient member is adhered to at least one pinch tab.

According to a further aspect, the resilient member is a foam.

According to a further aspect, the resilient member is an elastic band wrapped around the pair of arms.

According to a further aspect, the pair of arms each include a groove, the elastic band is received in the groove of each of the pair of arms.

According to a further aspect, the groove on each of the pair of arms is located between the hinge and the wing.

Further areas of applicability will become apparent from the description provided herein. The description and specific examples in this summary are intended for purposes of illustration only and are not intended to limit the scope of the present disclosure.

### BRIEF DESCRIPTION OF THE DRAWINGS

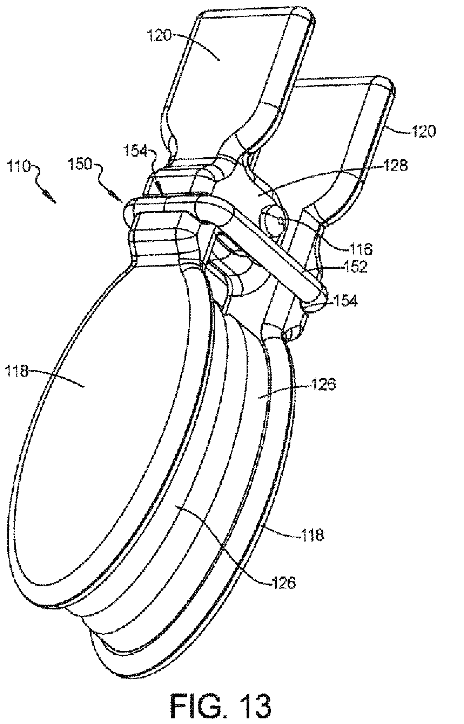

The drawings described herein are for illustrative purposes only of selected embodiments and not all possible implementations, and are not intended to limit the scope of the present disclosure. Figure 13 shows the invention.
FIG. 1 is a perspective view of the epistaxis clip according to the principles of the present disclosure;
FIG. 2 is a side plan view of the epistaxis clip according to the principles of the present disclosure shown in an open position;
FIG. 3 is a detailed perspective view of the locking mechanism of the epistaxis clip according to a first embodiment in an unlatched position;
FIG. 4 is a side plan view of the epistaxis clip according to the principles of the present disclosure shown in a closed position;
FIG. 5 is a detailed perspective view of the locking mechanism of the epistaxis clip according to a first embodiment in a latched position;
FIG. 6 is a detailed perspective view of the locking mechanism of the epistaxis clip according to a second embodiment in a partially latched position;
FIG. 7 is a detailed perspective view of the locking mechanism of the epistaxis clip according to a second embodiment in a partially latched position;
FIG 8 is a side plan view of an epistaxis clip according to a third embodiment in an un-latched position;
FIG. 9 is a side plan view of the epistaxis clip of FIG. 8 in a latched position;
FIG. 10 is a side plan view of an epistaxis clip according to a fourth embodiment in a latched position;
FIG. 11 is a side plan view of the epistaxis clip of FIG. 10 in an un-latched position;
FIG 12 is a front perspective view of an epistaxis clip according to a fifth embodiment; and
FIG. 13 is a front perspective view of an epistaxis clip according to a sixth embodiment.

Corresponding reference numerals indicate corresponding parts throughout the several views of the drawings.

### DETAILED DESCRIPTION

Example embodiments will now be described more fully with reference to the accompanying drawings.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

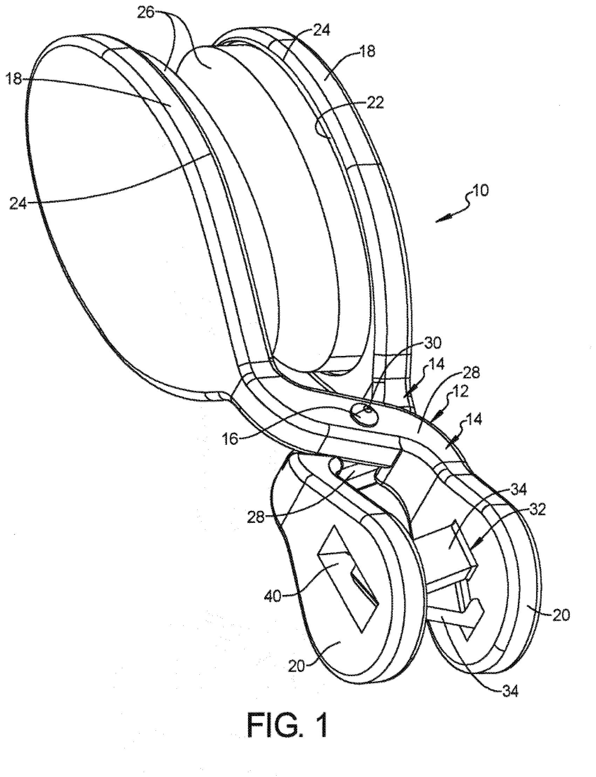

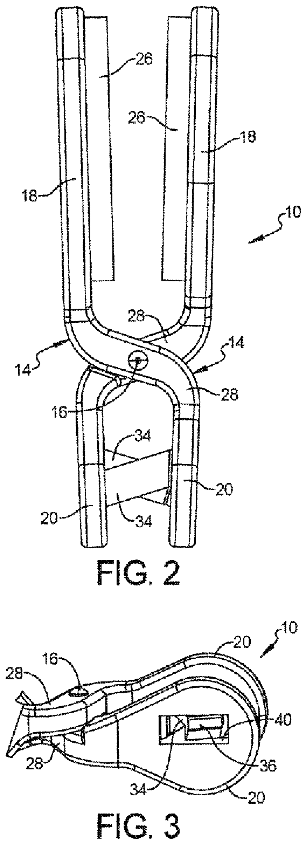

With reference to FIGS. 1-5, an epistaxis clip 10 is shown for applying predetermined pressure on the Kiesselbach's plexus in order to treat a nosebleed. The epistaxis clip 10 includes a frame structure 12 made from plastic or other engineering material. The frame structure 12 includes a pair of arms 14 that are connected to one another by a hinge pin 16. Each of the pair of arms 14 include a wing 18 at one end and a pinch tab 20 at another end. Although the wings 18 are shown as being oblong or paddle shaped, other shapes can be used.

The pair of wings 18 can each define a pair of opposing recesses 22 surrounded by a rib 24. A pair of absorbent pads 26 are disposed on the respective opposing pair of wings 18. The absorbent pads 26 can be adhered or press-fit in the recesses 22. As the absorbent pads 26 absorb blood, they are allowed to expand further into the recesses 22. The absorbent pads 26 can also be replaceable within the wings 18 through either adhesive or press-fit.

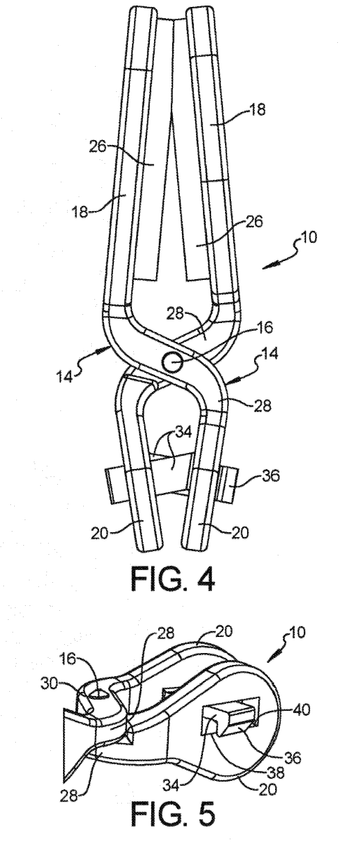

The pair of arms 14 include a curved body 28 connecting the wing 18 to the pinch tab 20. The curved body 28 of each arm can include an aperture 30 for receiving the hinge pin 16 for hingedly connecting the pair of arms 14 together. The pinch tabs 20 can be pressed toward one another in order to separate the pair of wings 18 away from one another. The pinch tabs 20 include a locking mechanism 32 for securing the pair of wings 18 in a clamping position within the nostrils and against a patient's septum. The locking mechanism 32 can include a latching arm 34 extending from one or both pinch tabs 20 and having a latch 36 that engages a ledge 38 of the opposing pinch tab 20, as shown in FIGS. 4 and 5. The ledge 38 can be disposed along an opening 40 in the pinch tabs 20. The end of the latching arm 34 can have a beveled end 42 that slides across a side face of the opening 40 until the latch 36 engages the ledge 38 for securing the epistaxis clip in a clamped position.

The absorbent pads 26 absorb blood at the site of the bleeding and can include a flexible silicone coated foam, a non-woven foam or a PVA that conform to the wound bed contouring and does not stick to the wound. The absorbent pads 26 can also be impregnated with Sodium Polyacrylate or other material for maximum absorption. In addition, the absorbent pads 26 can be provided with antimicrobial agents to avoid toxic shock syndrome and infection. The angle of the wings 18 applies compression at the Kiesselbach's plexus of the septum of at least 20 mmHg. When the bleeding has ceased, the epistaxis clip 10 can be removed from the nasal passages without fear of adhering to the wound or mucosa and can be discarded.

The pair of wings 18 of the epistaxis clip 10 are designed with a low profile so as not to occlude the nasal passages. The frame structure 12 can be formed from a clear plastic and extends from the nostrils below the columella. The ends of the latch arms 34 can be pinched inward by a user's fingers to release the epistaxis clip 10 from its clamped position.

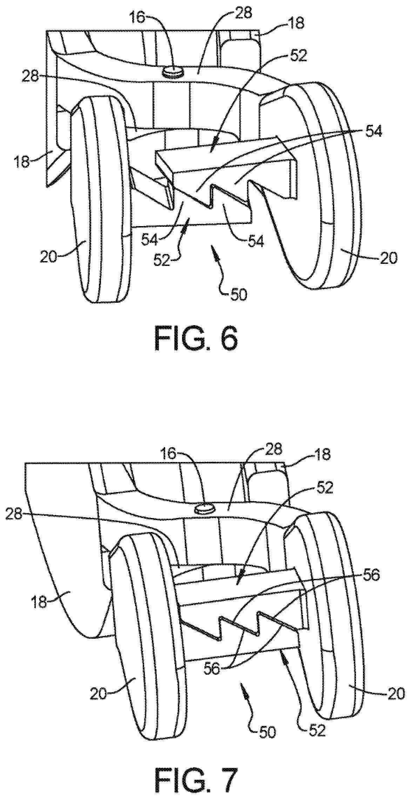

With reference to FIGS. 6 and 7, an alternative locking mechanism 50 will now be described. The locking mechanism 50 can include a pair of latching arms 52 extending from both pinch tabs 20 that each include a plurality of teeth 54 that engage each other to varying positions, as shown in FIGS. 6 and 7 so that the wings can be clamped to different tightnesses. The end of the latching arms 52 and the plurality of teeth 54 can have a beveled face 56 that slides across a beveled face of the opposing latching arms 52.

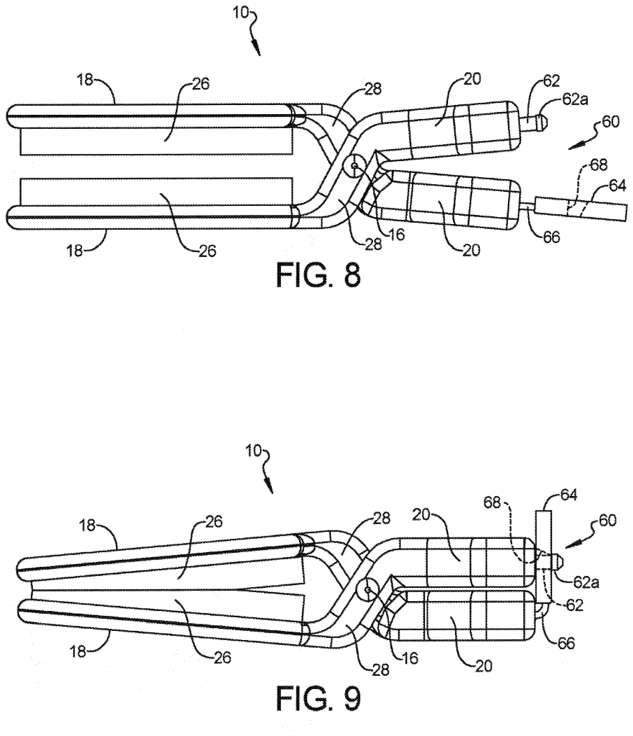

With reference to FIGS. 8 and 9, an alternative locking mechanism 60 will now be described. The locking mechanism 60 can include a pin 62 extending from one pinch tab 20 and a flap 64 supported by the other pinch tab by a living hinge 66, the flap having an aperture 68 that receives the pin 62. The flap 64 can be manipulated by the user to receive the pin 62 in the aperture 68 in order to hold the epistaxis clip 10 in a clamped position. The pin 62 can include a collar 62a at a distal end that latches the pin 62 within the aperture 68. The flap 64 and pin 62 lock the epistaxis clip in a clamped position.

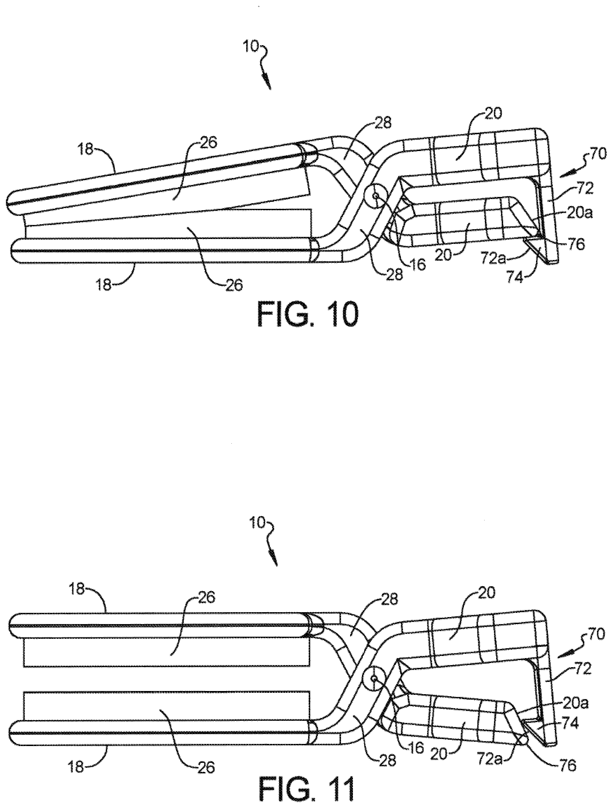

With reference to FIGS. 10 and 11, an alternative locking mechanism 70 will now be described. The locking mechanism 70 can include a latching arm 72 extending from one pinch tab 20 and including a latch portion 74 that engages the other pinch tab 20 to clamp the wings 18 in an engaged position within the user's nostrils. The end of the latching arm 72 and the other pinch tab 20 can each have a beveled face 72a, 20a, respectively that slides across one another until the latch portion 74 engages a ledge 76 of the other pinch tab 20 in order to hold the epistaxis clip 10 in a clamped position.

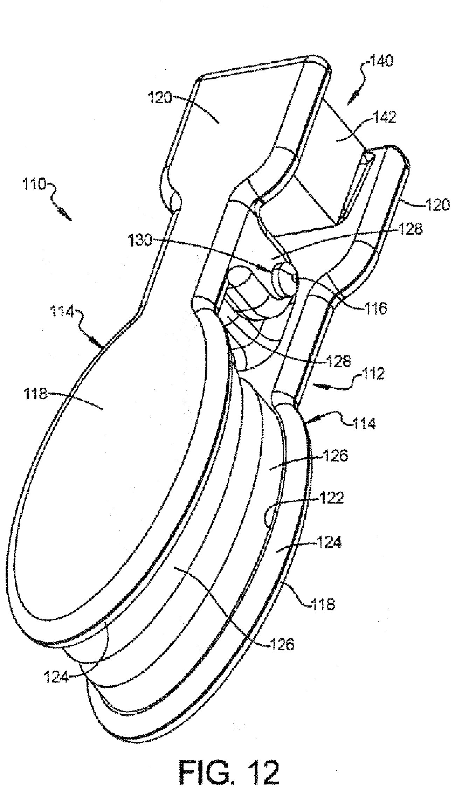

With reference to FIG. 12, an alternative epistaxis clip 110 is shown for applying predetermined pressure on the Kiesselbach's plexus in order to treat a nosebleed. The epistaxis clip 110 includes a frame structure 112 made from plastic or other engineering material. The frame structure 112 includes a pair of arms 114 that are connected to one another by a hinge pin 116. Each of the pair of arms 114 include a wing 118 at one end and a pinch tab 120 at another end.

The pair of wings 118 can each define a pair of opposing recesses 122 surrounded by a rib 124. A pair of absorbent pads 126 are disposed on the respective opposing pair of wings 118. The absorbent pads 126 can be adhered or press-fit in the recesses 122. As the absorbent pads 126 absorb blood, they are allowed to expand further into the recesses 122. The absorbent pads 126 can also be replaceable within the wings 118 through either adhesive or press-fit.

The pair of arms 114 include a protruding hinge portion 128. The hinge portion 128 of each arm 114 can include an aperture 130 for receiving the hinge pin 116 for hingedly connecting the pair of arms 114 together. The pinch tabs 120 can be pressed toward one another in order to separate the pair of wings 118 away from one another. The locking mechanism 140 can include a resilient body 142 disposed between the pinch tabs 120. The resilient body 142 can be adhered to or otherwise connected to one or both of the pinch tabs 120 and biases the pinch tabs 120 apart in order to bias the wings 118 toward a clamped position. The pinch tabs 120 can be squeezed toward one another by a user in order to separate the wings 118 away from one another for insertion into a patient's nostrils. When the pinch tabs 120 are released, the resilient body 142 biases the pinch tabs 120 apart and biases the wings 118 toward the clamped position to apply. The resilient body 142 can be a block of resilient material such as foam or silicone.

With reference to FIG. 13, a locking mechanism 150 to be used in an embodiment of the invention will now be described. The locking mechanism 150 includes a resilient band 152 wrapped around the arms 114 to bias the wings 118 toward a clamped position. The resilient band 152 can be made of a rubber or other elastic material. The pair of arms 114 each include a groove 154 located between the wings 118 and the hinge portions 128 for receiving the resilient band 152 therein. When the pinch tabs 120 are pinched together, the resilient band 152 is stretched to allow the wings 118 to be separated from one another to allow the wings 118 to be inserted in a patient's nostrils.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. An epistaxis clip (110), comprising:
a pair of arms (114) hingedly connected to one another, each pair of arms (114) include a wing (118) at a first end and a pinch tab (120) at a second end;
a pair of absorbent pads (126) is supported by the wing (118) of each of the pair of arms (114); **characterized in that**
the epistaxis clip (110) further comprising a resilient member (142) engaged with the pair of arms (114) for biasing the wings (118) to a clamped position;
and
the resilient member is an elastic band (152) wrapped around the pair of arms (114).

2. The epistaxis clip according to claim 1 wherein the pair of arms (114)
each include a groove (154), the elastic band (152) is received in the groove (154) of each of the pair of arms (114).

3. The epistaxis clip according to claim 2 wherein the groove (154) on each of the pair of arms (114) is located between the hinge and the wing (118).

## Patentansprüche

1. Epistaxisklammer (110), umfassend:
ein Paar Arme (114), die durch ein Scharnier miteinander verbunden sind, wobei jedes Paar Arme (114) einen Flügel (118) an einem ersten Ende und eine Klemmlasche (120) an einem zweiten Ende umfasst;
wobei ein Paar saugfähiger Kissen (126) durch den Flügel (118) jedes des Paars Arme (114) gehalten wird; **dadurch gekennzeichnet, dass**
die Epistaxisklammer (110) ferner ein elastisches Element (142) umfasst, das mit dem Paar Arme (114) in Eingriff steht, um die Flügel (118) in eine eingespannte Position vorzuspannen;
und
das elastische Element ein elastisches Band (152) ist, das um das Paar Arme (114) gewickelt ist.

2. Epistaxisklammer nach Anspruch 1, wobei das Paar Arme (114) jeweils eine Nut (154) umfasst, wobei das elastische Band (152) in der Nut (154) jedes des Paars Arme (114) aufgenommen ist.

3. Epistaxisklammer nach Anspruch 2, wobei sich die Nut (154) auf jedem des Paars Arme (114) zwischen dem Scharnier und dem Flügel (118) befindet.

## Revendications

1. Pince d'épistaxis (110), comprenant :
une paire de bras (114) reliés par charnière l'un à l'autre, chaque paire de bras (114) comprenant une aile (118) au niveau d'une première extrémité et une patte de pincement (120) au niveau d'une seconde extrémité ;
une paire de tampons absorbants (126) est supportée par l'aile (118) de chacun de la paire de bras (114) ; **caractérisée en ce que**
la pince d'épistaxis (110) comprenant en outre un élément élastique (142) en prise avec la paire de bras (114) afin de solliciter les ailes (118) vers une position serrée ; et
l'élément élastique est une bande élastique (152) enroulée autour de la paire de bras (114).

2. Pince d'épistaxis selon la revendication 1, la paire de bras (114) comprenant chacun une rainure (154), la bande élastique (152) étant reçue dans la rainure (154) de chacun de la paire de bras (114).

3. Pince d'épistaxis selon la revendication 2, la rainure (154) sur chacun de la paire de bras (114) étant située entre la charnière et l'aile (118).
